**Europäisches Patentamt**

**European Patent Office**

**Office européen des brevets**

(19)

(11) Publication number : **0 255 827 B1**

# (12) EUROPEAN PATENT SPECIFICATION

(45) Date of publication of patent specification :
**18.03.92 Bulletin 92/12**

(51) Int. Cl.⁵ : **A61K 9/20**

(21) Application number : **87900931.4**

(22) Date of filing : **13.01.87**

(86) International application number :
**PCT/US87/00031**

(87) International publication number :
**WO 87/04342 30.07.87 Gazette 87/17**

(54) **BUCCAL FORMULATION.**

(30) Priority : **22.01.86 US 821358**

(43) Date of publication of application :
**17.02.88 Bulletin 88/07**

(45) Publication of the grant of the patent :
**18.03.92 Bulletin 92/12**

(84) Designated Contracting States :
**AT BE CH DE FR GB IT LI LU NL SE**

(56) References cited :
**EP-A- 0 095 944**
**FR-A- 2 285 896**
**US-A- 3 033 754**
**Journal of Pharmaceutical Sciences, volume 62, no. 1 1, January 1973, (Washington, US), S.S. Kornblum et al.: " A new table disintegrating agent: crosslinked polyvinylpyrrolidone ", pages 43-49, see page 45, table 1**

(73) Proprietor : **Key Pharmaceuticals, Inc.**
**2000 Galloping Hills Road**
**Kenilworth New Jersey 07033 (US)**

(72) Inventor : **HSIAO, Chiin, H.**
**4890 S.W. 104 Avenue**
**Cooper City, FL 33328 (US)**
Inventor : **CACACE, Janice, L.**
**615 S.W. 12 Street**
**Gainesville, FL 32601 (US)**
Inventor : **McCARTY, John, Alexander**
**630 N.E. 121 Street**
**Miami, FL 33161 (US)**

(74) Representative : **Werner, Hans-Karsten, Dr. et al**
**Deichmannhaus am Hauptbahnhof**
**W-5000 Köln 1 (DE)**

## Description

This application is directed to a formulation for the buccal administration of an active ingredient. Buccal administration (in the pouch of the cheek of the subject) is particularly useful for active ingredients which show poor bioavailability upon administration through other non-parenteral modes. This poor availability can be attributed to low solubility, degradation by enzyme or destruction by acid upon passing through the intestinal tract, or first-pass destruction by the liver after absorption from the gastrointestinal tract. Examples of such medicaments include: steroids such as estrogens, e.g. estradiol and derivatives such as its esters, for example the valerate, cypionate and propionate, progestins, e.g., progesterone and related compounds, androgens and anabolic steroids; propranolol; thyroid hormones; pH-sensitive peptides and small proteins such as insulin and ACTH; physostigmine; scopolamine; verapamil; and gallopamil. It is also possible to administer compounds having good oral bioavailability buccally, but normally such medicaments would be administered orally for convenience.

FR-A-2 185 896 relates to preparations for administration by the oral cavity and is based on the use of sodium polyacrylate (PANA) in an oral preparation. The stated advantage of sodium polyacrylate in a buccal preparation is that it adheres strongly to a local site in the oral cavity and dissolves gradually over a period of time while releasing the active ingredient.

EP-A-95 944 relates to a pharmaceutical composition of an anti-androgenically active compound for use in the oral cavity. The preparation comprises conventionally "formulating materials including release controller, vehicle, binder, lubricant, absorber and corrigent etc." (see page 5, line 4 - 5 and page 7, line 7 - 8).

The Journal of Pharm. Science 62, 43 - 49, is a general discussion of the use of cross-linked polyvinylpyrrolidone as a tablet disintegrating agent. The present invention is not directed to specific use of cross-linked polyvinylpyrrolidone as a disintegrating agent in any pharmaceutical composition.

Buccal administration of estradiol gives an early peak in the blood level followed by decreasing concentration. This tracks the natural occurrence of estradiol in the body, and thus is an improvement over transdermal administration, which provides a relatively constant blood level. Oral administration of estrogens such as estradiol is unpractical in view of the destruction of the active ingredient in the liver shortly after absorption from the gastrointestinal tract.

It is necessary for a buccal formulation to remain in contact with the oral mucosa for a time sufficient for absorption of the medicament that is being administered. If the formulation falls apart too quickly, the active ingredient is swallowed, and an insufficient amount of medicament is delivered. If the formulation does not fall apart quickly enough, difficulties in patient compliance can result, since the patient should not each or drink while using the buccal formulation. The formulation should be of a small size to avoid discomfort to the patient, and it is desirable that as much of the formulation as possible be soluble in saliva so that discomfort in the form of insoluble gritty particles in the mouth-can be avoided.

This invention provides a buccal composition for administration of a medicament, comprising, as essential ingredients: 1 to 20% by weight of a soluble, pharmaceutically acceptable polymeric adhesive selected from the group consisting of acrylic acid polymer known as

    – carbomers (molecular weight 450,000 to 4,000,000),
    – partially hydrolyzed (87 - 89%) polyvinylalcohol (molecular weight 10,000 to 115,000),
    – polyethylene oxide (molecular weight 100,000 to 5,000,000),
    – hydroxypropyl methylcellulose (molecular weight 13,000 to 140,000),
    – hydroxypropyl cellulose (molecular weight 60,000 to 1,000,000);

up to 10% weight of pharmaceutically acceptabel tablet disintegrant; a soluble, directly compressible tablet excipient; and a therapeutically useful amount of medicament. The composition, which is in unit dosage form, contains up to 10% (e.g., 1 to 10%) by weight of a pharmaceutically acceptable tablet disintegrant.

According to a further feature, this invention is directed to a buccal composition in unit dosage form for administration of an estrogen, comprising 2 to 10% by weight of polymeric adhesive, e.g. carbomer 934 P; up to about 6% by weight tablet disintegrant, e.g. crospovidone; compressible sugar; and 50 micrograms to 2 mg of estradiol.

The buccal formulation of the present invention can also contain incidental ingredients, for example lubricants, coloring agents and flavoring agents.

The soluble, pharmaceutically acceptable polymeric adhesive is used to provide tackiness to the buccal formulation so that it will be held in place upon administration. The amount of adhesive in the formulation is 1-20% by weight, preferably 2-10%. Use of amounts less than 1% may result in insufficient adhesive properties or the formulation falling apart too quickly, whereas excessive amounts may result in the formulation lasting for a longer period than is desirable. The adhesives desirably are sticky when moist but not when dry, for convenience in handling. The amount of adhesive which can be used generally increases with the solubility of the active ingredient.

One particularly desirable group of polymeric adhesives is high molecular weight polymers of acrylic acid known as carbomers. Molecular weights of 450,000 to 4,000,000 are particularly useful, especially about 3,000,000 (e.g., as for carbomer 934 P). These substances are sold by B.F. Goodrich under the trademark Carbopol . These adhesives have been found to allow use of very small amounts to provide the desired adhesive characteristics to the formulation, which is advantageous since large amounts of adhesive may impede the dissolution of the active ingredient. Other suitable hydrophilic polymers usable as polymeric adhesives include partially (e.g. 87-89%) hydrolyzed polyvinylalcohol (molecular weight 10,000 to 125,000, preferably 11,000 to 31,000), polyethylene oxide (molecular weight 100,000 to 5,000,000, preferably about 400,000).

Hydroxypropyl methylcellulose, having a molecular weight of 13,000 to 140,000 (sold under the trademark Methocel by Dow), and hydroxypropyl cellulose, having a molecular weight of 60,000 to 1,000,000 (sold under the trademark Klucel ) also are useful adhesives. Material toward the high end of each of the molecular weight ranges is preferred. The term "soluble" is used throughout this application as an indication that the material is soluble in water or saliva.

Upon administration of the formulation, the adhesive therein forms a gel-like substance which is gradually broken up. Use of a small amount of a pharmaceutically acceptable disintegrant that swells upon administration, thus exposing more of the formulation to saliva, can aid this break-up and cause the formulation to break up gradually. The amount of disintegrant in the formulation is up to 10% by weight, e.g., 3-6%. However, excessive amounts of disintegrant actually may unduly delay disintegration, as by formation of an insoluble gel, instead of aiding dissolution of the formulation by expansion. Indeed, some formulations of this type may show faster disintegration if less than 3%, e.g., 1.5%, or even only 1% or less, disintegrant is used, especially where the disintegrant is substantially non-wettable by water or sparingly soluble in water; such a disintegrant indeed by inhibiting entry of water into the composition can unduly delay its disintegration and dissolution. The selection of the right amount of disintegrant can nonetheless be found by trial and error. Some formulations may lack disintegrant altogether or contain only a very small percentage, e.g. 0.05% or 0.1% to 0.9%.

One useful disintegrant is the material crospovidone, which is a cross-linked polyvinylpyrrolidone product. This material is sold under the trademark Polyplasdone XL by GAF. Other useful disintegrants include Ac-di-sol (FMC's trademark for croscarmellose, a cross-linked carboxymethylcellulose), alginic acid and sodium carboxymethyl starch such as that sold as Explotab by Edward Mendell Co., Inc.

The formulation also includes a soluble, directly compressible tableting excipient such as a sugar. One such useful tableting excipient is a co-crystallization of 97% sucrose and 3% highly modified dextrins sold under the trademark Di-Pac by Amstar. Other such excipients known to those skilled in the art, such as lactose, also may be used. The amount of excipient used is such that the resulting formulation is big enough to be handled conveniently, yet small enough to dissolve properly. Other ingredients which may be used include lubricants, coloring agents and flavoring agents. The lubricant may be water-insoluble, e.g. magnesium stearate or oleate, conveniently in an amount of up to 3.0% by weight, preferably 0.3% to 1.5%. However, a preferred lubricant is water-soluble, e.g. sodium lauryl sulfate, conveniently in an amount of up to 3.0% by weight, preferably 0.3% to 1.5%. A mixture of water-soluble and water-insoluble lubricants can be used. A soluble lubricant may tend to shorten disintegration and dissolution times, especially for a water-insoluble medicament, -whereas an insoluble lubricant may tend to lengthen them.

Active ingredients useful with this invention include those mentioned in the first paragraph of this specification. Of course, the amount will vary depending upon the dosage desired for a given treatment. Estradiol, when used as the active ingredient, is conveniently present in the amount of about 50 micrograms to about 2 mg.

The formulations of the present invention can be prepared by simply mixing the ingredients together and compressing desired amounts of the mixture into tablet form. The final formulations desirably have a diameter of about 0.635 cm (about a quarter inch) and a thickness of about 0.127 cm (about 0.05 inches), and upon administration disintegrate in 2-20 minutes, preferably 4-12 minutes.

The present invention is illustrated by the following examples.

EXAMPLE 1

The following ingredients are charged into a blender and mixed for ten minutes.

| % BY WEIGHT | INGREDIENT | AMOUNT |
|---|---|---|
| 0.2 | Estradiol, USP (Micronized) | 2.0 g |
| 89.3 | Di-Pac (Compressible Sugar, NF) | 893.0 g |
| 5.0 | Carbomer 934P, NF (Carbopol 934P) | 50.0 g |
| 5.0 | Crospovidone, NF (Polyplasdone XL) | 50.0 g |
| 0.5 | Magnesium Stearate, NF | 5.0 g |
| 100.0 | | 1,000.0 g |

Tablets weighing about 0.05 g each are formed using a compression force of about 6,900 kPa (about 1000 PSI). The batch yields about 20,000 tablets which upon administration disintegrate in 10-15 minutes. The tablets are about 0.635 cm (about a quarter inch) in diameter.

EXEMPLE 2

Following the procedure of Example 1, the following are mixed and formed into tablets:

| % BY WEIGHT | INGREDIENT | AMOUNT |
|---|---|---|
| 0.4 | Estradiol, USP (Micronized) | 4.0 g |
| 89.0 | Di-Pac (Compressible Sugar, NF) | 890.0 g |
| 5.0 | Carbomer 934P, NF (Carbopol 934P) | 50.0 g |
| 5.0 | Crospovidone, NF (Polyplasdone XL) | 50.0 g |
| 0.5 | Magnesium Stearate, NF | 5.0 g |
| 0.1 | FDC Yellow #6 Lake | 1.0 g |
| 100.0 | | 1,000.0 g |

Results similar to those of Example 1 are obtained.

EXEMPLE 3

Following the procedure of Example 1, the following are mixed and formed into tablets:

| % BY WEIGHT | INGREDIENT | AMOUNT |
|---|---|---|
| 0.2 | Estradiol, USP (Micronized) | 2.0 g |
| 96.3 | Di-Pac (Compressible Sugar, NF) | 963.0 g |
| 2.5 | Carbomer 934P, NF (Carbopol 934P) | 25.0 g |
| 1.0 | Sodium lauryl sulfate NF | 10.0 g |
| 100.0 | | 1,000.0 g |

These tablets disintegrate in 3 to 3-and-a-half minutes, but dissolution is complete only after about 45 minutes.

EXEMPLE 4

Following the procedure of Example 1, the following are mixed and formed into tablets:

| % BY WEIGHT | INGREDIENT | AMOUNT |
|---|---|---|
| 0.2 | Estradiol, USP (Micronized) | 2.0 g |
| 96.2 | Di-Pac (Compressible Sugar, NF) | 962.0 g |
| 2.5 | Carbomer 934P, NF (Carbopol 934P) | 25.0 g |
| 0.1 | Crospovidone, NF (Polyplasdone XL) | 1.0 g |
| 1.0 | Sodium lauryl sulfate, NF | 10.0 g |
| 100.0 | | 1,000.0 g |

EXAMPLE 5

Following the procedure of Example 1, the following are mixed and forced into tablets:

| % BY WEIGHT | INGREDIENT | AMOUNT |
|---|---|---|
| 0.2 | Estradiol, USP (Micronized) | 2.0 g |
| 95.4 | Di-Pac (Compressible Sugar, NF) | 954.0 g |
| 2.5 | Carbomer 934P, NF (Carbopol 934P) | 25.0 g |
| 0.9 | Crospovidone, NF (Polyplasdone XL) | 9.0 g |
| 1.0 | Sodium lauryl sulfate, NF | 10.0 g |
| 100.0 | | 1,000.0 g |

The estradiol used in the above Examples can be replaced with appropriate amounts of other active ingredients, e.g., those mentioned in the first paragraph of this specification, with appropriate adjustment of the amount of other ingredients, especially of the compressible sugar.

**Claims**

**Claims for the following Contracting States: BE, CH, DE, FR, GB, IT, LU, NL, SE**

1. A buccal composition for administration of an active ingredien, which upon administration disintegrates in 2-20 minutes, comprising as essential ingredients:
   (a) 1 to 20% by weight of a soluble, pharmaceutically acceptable polymeric adhesive selected from the group consisting of acrylic acid polymer known as
      – carbomers (molecular weight 450,000 to 4,000,000),
      – partially hydrolyzed (87 - 89%) polyvinylalcohol (molecular weight 10,000 to 125,000),
      – polyethylene oxide (molecular weight 100,000 to 5,000,000),
      – hydroxypropyl methylcellulose (molecular weight 13,000 to 140,000),
      – hydroxypropyl cellulose (molecular weight 60,000 to 1,000,000);
   (b) up to 10% by weight of a pharmaceutically acceptable tablet disintegrant
   (c) a soluble, directly compressible tablet excipient; and
   (d) a therapeutically useful amount of active ingredient.
2. A composition as claimed in claim 1 wherein said excipient is a compressible sugar.
3. A composition as claimed in any of claims 1 or 2 wherein said active ingredient is a progestin, an androgen, an anabolic steroid, propranolol, insulin, ACTH, physostigmine, scopolamine, verapamil, or gallopamil, or especially an estrogen, in particular estradiol or a pharmaceutically acceptable derivative thereof, which is preferably present in an amount of 50 micrograms to 2 mg.
4. A composition as claimed in any of claims 1 to 3 further comprising 3 to 6% by weight, of a pharmaceutically acceptable tablet disintegrant,
5. A composition as claimed in claim 3 wherein said disintegrant is cross-linked polyvinylpyrrolidone, crosslinked carboxymethylcellulose, alginic acid or sodium carboxymethyl starch, especially crospovidone.

6. A composition as claimed in claim 4 or claim 5 wherein the amount of disintegrant is less than 1% by weight.

7. A buccal composition as claimed in claim 1 for unit dosage administration of an estrogen, comprising:

(a) 2 to 10% by weight of carbomer 934P;

(b) 3 to 6% by weight crospovidone;

(c) compressible sugar; and

(d) a therapeutically useful amount of an estrogen.

8. A buccal composition as claimed in claim 1 for unit dosage administration of an estrogen, comprising:

(a) 2 to 10% by weight of carbomer 934p;

(b) less than 1% by weight crospovidone;

(c) compressible sugar; and

(d) a therapeutically useful amount of an estrogen.

9. A composition as claimed in claim 7 or claim 8 wherein the estrrogen is estradiol in the amount of 50 micrograms to 2 mg. per unit dose.

## Claims for the following Contracting State: AT

1. Process for preparing a buccal composition for administration of an active ingredient, which upon administration disintegrates in 2 - 20 minutes, comprising as essential ingredients:

(a) 1 to 20% by weight of a soluble, pharmaceutically acceptable polymeric adhesive selected from the group consisting of acrylic acid polymer known as

- carbomers (molecular weight 450,000 to 4,000,000),
- partially hydrolyzed (87 - 89%) polyvinylalcohol (molecular weight 10,000 to 125,000),
- polyethylene oxide (molecular weight 100,000 to 5,000,000),
- hydroxypropyl methylcellulose(molecular weight 13,000 to 140,000),
- hydroxypropyl cellulose(molecular weight 60,000 to 1,000,000);

(b) up to 10% by weight of a pharmaceutically acceptable tablet disintegrant

(c) a soluble, directly compressible tablet excipient; and

(d) a therapeutically useful amount of active ingredient, characterized in that thze ingredients are mixed together.

2. Process as claimed in claim 1 wherein said excipient is a compressible sugar.

3. Process as claimed in any of claims 1 or 2 wherein said active ingredient is a progestin, an androgen, an anabolic steroid, propranolol, insulin, ACTH, physostigmine, scopolamine, verapamil, or gallopamil, or especialy an estrogen, in particular estradiol or a pharmaceutically acceptable derivative thereof, which is preferably present in an amount of 50 micrograms to 2 mg.

4. Process as claimed in any of claims 1 to 3 further comprising 3 to 6% by weight, of a pharmaceutically acceptable tablet disintegrant,

5. Process as claimed in claim 3 wherein said disintegrant is cross-linked polyvinylpyrrolidone, crosslinked carboxymethylcellulose, alginic acid or sodium carboxymethyl starch, especially crospovidone.

6. Process as claimed in claim 4 or claim 5 wherein the amount of disintegrant is less than 1% by weight.

7. Process as claimed in claim 1 for nit dosage administration of an estrogen, comprising:

(a) 2 to 10% by weight of carbomer 934P;

(b) 3 to 6% by weight crospovidone;

(c) compressible sugar; and

(d) a therapeutically useful amount of an estrogen.

8. Process as claimed in claim 1 for unit dosage administration of an estrogen, comprising:

(a) 2 to 10% by weight of carbomer 934P;

(b) less than 1% by weight crospovidone;

(c) compressible sugar; and

(d) a therapeutically useful amount of an estrogen.

9. Process as claimed 7 or claim 8 wherein the estrogen is estradiol in the amount of 50 micrograms to 2 mg. per unit dose.

**Patentansprüche**

**Patentanspruch für folgenden Vertragsstaaten: BE, CH, DE, FR, GB, IT, LU, NL, SE**

1. Buccale Zusammensetzung zur Verabreichung eines wirksamen Bestandteils, die auf Verabreichung in 2 - 20 min zerfällt, als wesentliche Bestandteile umfassend:
   a) 1 bis 20 Gew.-% eines löslichen, pharmazeutisch annehmbaren, polymeren Klebemittels, ausgewählt aus der Gruppe bestehend aus Acrylsäure-Polymer, geläufig als
   – Carbomere (Molekulargewicht 450 000 bis 4 000 000),
   – teilweise hydrolysierter (87-89%) Polyvinylalkohol (Molekulargewicht 10 000 bis 125 000),
   – Polyethylenoxid (Molekulargewicht 100 000 bis 5 000 000),
   – Hydroxypropylmethylcellulose (Molekulargewicht 13 000 bis 140 000),
   – Hydroxypropylcellulose (Molekulargewicht 60 000 bis 1 000 000);
   b) bis zu 10 Gew.-% eines pharmazeutisch annehmbaren Tablettenauflösemittels,
   c) einen löslichen, direkt preßbaren Tablettenfüllstoff; und
   d) eine therapeutisch brauchbare Menge an wirksamem Bestandteil.

2. Zusammensetzung nach Anspruch 1, wobei der Füllstoff ein preßbarer Zucker ist.

3. Zusammensetzung nach irgendeinem der Ansprüche 1 oder 2, wobei der wirksame Bestandteil ein Progesteron ist, ein Androgen, ein anaboles Steroid, Propranolol, Insulin, ACTH, Physostigmin, Scopolamin, Verapamil oder Gallopamil, oder besonders ein Estrogen, insbesondere Estradiol oder ein pharmazeutisch annehmbares Derivat davon, das vorzugsweise in einer Menge von 50 µg bis 2 mg vorhanden ist.

4. Zusammensetzung nach irgendeinem der Ansprüche 1 bis 3, des weiteren umfassend 3 bis 6 Gew.-% eines pharmazeutisch annehmbaren Tablettenauflösemittels.

5. Zusammensetzung nach Anspruch 3, wobei das Auflösemittel vernetztes Polyvinylpyrrolidon ist, vernetzte Carboxymethylcellulose, Alginsäure oder Natrium-carboxymethyl-Stärke, besonders Crospovidon.

6. Zusammensetzung nach Anspruch 4 oder 5, wobei sich die Menge an Auflösemittel auf weniger als 1 Gew.-% beläuft.

7. Buccale Zusammensetzung nach Anspruch 1 zur Verabreichung eines Estrogens als Einheitsdosierung, umfassend:
   a) 2 bis 10 Gew.-% Carbomer 934P;
   b) 3 bis 6 Gew.-% Crospovidon;
   c) preßbaren Zucker; und
   d) eine therapeutisch brauchbare Menge eines Estrogens.

8. Buccale Zusammensetzung nach Anspruch 1 zur Verabreichung eines Estrogens als Einheitsdosierung, umfassend:
   a) 2 bis 10 Gew.-% Carbomer 934P;
   b) weniger als 1 Gew.-% Crospovidon;
   c) preßbaren Zucker; und
   d) eine therapeutisch brauchbare Menge eines Estrogens.

9. Zusammensetzung nach Anspruch 7 oder 8, wobei das Estrogen Estradiol ist, in einer Menge von 50 µg bis 2 mg je Einheitsdosis.

**Patentanspruch für folgenden Vertragsstaat: AT**

1. Verfahren zur Herstellung einer buccalen Zusammensetzung zur Verabreichung eines wirksamen Bestandteils, die auf Verabreichung in 2 - 20 min zerfällt, als wesentliche Bestandteile umfassend:
   a) 1 bis 20 Gew.-% eines löslichen, pharmazeutisch annehmbaren, polymeren Klebemittels, ausgewählt aus der Gruppe bestehend aus Acrylsäure-Polymer, geläufig als
   – Carbomere (Molekulargewicht 450 000 bis 4 000 000),
   – teilweise hydrolysierter (87-89%) Polyvinylalkohol (Molekulargewicht 10 000 bis 125 000),
   – Polyethylenoxid (Molekulargewicht 100 000 bis 5 000 000),
   – Hydroxypropylmethylcellulose (Molekulargewicht 13 000 bis 140 000),
   – Hydroxypropylcellulose (Molekulargewicht 60 000 bis 1 000 000);
   b) bis zu 10 Gew.-% eines pharmazeutisch annehmbaren Tablettenauflösemittels,
   c) einen löslichen, direkt preßbaren Tablettenfüllstoff; und
   d) eine therapeutisch brauchbare Menge an wirksamem Bestandteil, dadurch gekennzeichnet, daß die Bestandteile zusammengemischt werden.

2. Verfahren nach Anspruch 1, wobei der Füllstoff ein preßbarer Zucker ist.

3. Verfahren nach irgendeinem der Ansprüche 1 oder 2, wobei der wirksame Bestandteil ein Progesteron ist, ein Androgen, ein anaboles Steroid, Propranolol, Insulin, ACTH, Physostigmin, Scopolamin, Verapamil oder Gallopamil, oder besonders ein Estrogen, insbesondere Estradiol oder ein pharmazeutisch annehmbares Derivat davon, das vorzugsweise in einer Menge von 50 µg bis 2 mg vorhanden ist.

4. Verfahren nach irgendeinem der Ansprüche 1 bis 3, des weiteren umfassend 3 bis 6 Gew.-% eines pharmazeutisch annehmbaren Tablettenauflösemittels.

5. Verfahren nach Anspruch 3, wobei das Auflösemittel vernetztes Polyvinylpyrrolidon ist, vernetzte Carboxymethylcellulose, Alginsäure oder Natrium-carboxymethyl-Stärke, besonders Crospovidon.

6. Verfahren nach Anspruch 4 oder 5, wobei sich die Menge an Auflösemittel auf weniger als 1 Gew.-% beläuft.

7. Verfahren nach Anspruch 1 zur Verabreichung eines Estrogens als Einheitsdosierung, umfassend:
a) 2 bis 10 Gew.-% Carbomer 934P;
b) 3 bis 6 Gew.-% Crospovidon;
c) preßbaren Zucker; und
d) eine therapeutisch brauchbare Menge eines Estrogens.

8. Verfahren nach Anspruch 1 zur Verabreichung eines Estrogens als Einheitsdosierung, umfassend:
a) 2 bis 10 Gew.-% Carbomer 934P;
b) weniger als 1 Gew.-% Crospovidon;
c) preßbaren Zucker; und
d) eine therapeutisch brauchbare Menge eines Estrogens.

9. Verfahren nach Anspruch 7 oder 8, wobei das Estrogen Estradiol ist, in einer Menge von 50 µg bis 2 mg je Einheitsdosis.

## Revendications

### Revendications pour les Etats contractants suivants: BE, CH, DE, FR, GB, IT, LU, NL, SE

1. Composition buccale pour une administration d'un ingrédient actif qui,lors de l'administration se désintègre en 2-20 minutes, comprenant comme ingrédients essentiels :
(a) 1 à 2-20 en poids d'un adhésif polymérique soluble, pharmaceutiquement acceptable, choisi dans le groupe consistant en polymère d'acide acrylique connu comme
carbomères (poids moléculaire 450.000 à 4.000.000),
alcool polyvinylique partiellement hydrolysé (87-89%) (poids moléculaire 10.000 à 125.000),
oxyde de polyéthylène (poids moléculaire 100.000 à 5.000.000),
hydroxypropyl méthylcellulose (poids moléculaire 13.000 à 140.000),
hydroxypropyl cellulose (poids moléculaire 60.000 à 1.000.000) ;
(b) jusqu'à 10% en poids d'un désintégrant de comprimés acceptable en pharmacie ;
(c) un excipient soluble, directement compressible de comprimé ; et
(d) une quantité thérapeutiquement utile de l'ingrédient actif.

2. Composition selon la revendication 1 où ledit excipient est un sucre compressible.

3. Composition selon l'une quelconque des revendications 1 ou 2 où ledit ingrédient actif est une progestine, un androgène, un stéroïde anabolique, un propanolol, une insuline, une ACTH, une physostigmine, une scopolamine, un vérapamil ou un gallopamil, ou en particulier un oestrogène, en particulier l'estradiol ou son dérivé acceptable en pharmacie, qui est de préférence présent à la quantité de 50 microgrammes à 2 mg.

4. Composition selon l'une quelconque des revendications 1 à 3 caractérisée en ce qu'elle comprend de plus 3 à 6% en poids d'un désintégrant de comprimés acceptable en pharmacie.

5. Composition selon la revendication 3 où le désintégrant est de la polyvinylpyrrolidone réticulée, de la carboxyméthylcellulose réticulée, de l'acide alginique ou de l'amidon de sodium carboxyméthyle, en particulier la crospovidone.

6. Composition selon la revendication 4 ou la revendication 5 où la quantité du désintégrant est inférieure à 1% en poids.

7. Composition buccale selon la revendication 1 pour une administration par dose unitaire d'un oestrogène, comprenant :
(a) 2 à 10% en poids de carbomère 934P ;
(b) 3 à 6% en poids de crospovidone ;
(c) un sucre compressible ; et
(d) une quantité thérapeutiquement utile d'un oestrogène.

8. Composition buccale selon la revendication 1 pour une administration par dose unitaire d'un oestrogène, comprenant :

(a) 2 à 10% en poids de carbomère 934P ;

(b) moins de 1% en poids de crospovidone ;

(c) un sucre compressible ; et

(d) une quantité thérapeutiquement utile d'un oestrogène.

9. Composition selon la revendication 7 ou la revendication 8 où l'oestrogène est l'estradiol à la quantité de 50 microgrammes à 2 mg par dose unitaire.

**Revendications pour l'Etat contractant suivant: AT**

1. Procédé de préparation d'une composition buccale pour l'administration d'un ingrédient actif, qui lors de l'administration se désintègre en 2-20 minutes, comprenant, comme ingrédients essentiels :

(a) 1 à 20% en poids d'un adhésif polymérique soluble, pharmaceutiquement acceptable, choisi dans le groupe consistant en polymère d'acide acrylique connu comme

– carbomères (poids moléculaires 450.000 à 4.000.000),

– alcool polyvinylique partiellement hydrolysé (87-89%) (poids moléculaire 10.000 à 125.000),

– oxyde de polyéthylène (poids moléculaire 100.000 à 5.000.000),

– hydroxypropyl méthylcellulose (poids moléculaire 13.000 à 140.000),

– hydroxypropyl cellulose (poids moléculaire 60.000 à 1.000.000) ;

(b) jusqu'à 10% en poids d'un désintégrant de comprimés acceptable en pharmacie ;

(c) un excipient soluble, directement compressible de comprimé ; et

(d) une quantité thérapeutiquement utile de l'ingrédient actif, caractérisé en ce que ces ingrédients sont mélangés ensemble.

2. Procédé selon la revendication 1 où ledit excipient est un sucre compressible.

3. Procédé selon l'une quelconque des revendications 1 ou 2 où ledit ingrédient actif est une progestine, un androgène, un stéroïde anabolique, un propanolol, une insuline, une ACTH, une physostigmine, une scopolamine, un vérapamil ou un gallopamil, ou en particulier un oestrogène, en particulier l'estradiol ou son dérivé acceptable en pharmacie, qui est de préférence présent à la quantité de 50 microgrammes à 2 mg.

4. Procédé selon l'une quelconque des revendications 1 à 3 comprenant de plus 3 à 6% en poids d'un désintégrant de comprimés acceptable en pharmacie.

5. Procédé selon la revendication 3 où ledit désintégrant est de la polyvinylpyrrolidone réticulée, de la carboxyméthylcellulose réticulée, de l'acide alginique ou de l'amidon de sodium carboxyméthyle, en particulier la crospovidone.

6. Procédé selon la revendication 4 ou la revendication 5 où la quantité du désintégrant est inférieure à 1% en poids.

7. Procédé selon la revendication 1 pour l'administration d'une dose unitaire d'un oestrogène, comprenant :

(a) 2 à 10% en poids de carbomère 934P ;

(b) 3 à 6% en poids de crospovidone ;

(c) un sucre compressible ; et

(d) une quantité thérapeutiquement utile d'un oestrogène.

8. Procédé selon la revendication 1 pour l'administration par dose unitaire d'un oestrogène, comprenant :

(a) 2 à 10% en poids de carbomère 934 ;

(b) moins de 1% en poids de crospovidone ;

(c) un sucre compressible ; et

(d) une quantité thérapeutiquement utile d'un oestrogène.

9. Procédé selon la revendication 7 ou la revendication 8 où l'oestrogène est l'estradiol à la quantité de 50 microgrammes à 2 mg par dose unitaire.